# EUROPEAN PATENT SPECIFICATION

(11) **EP 1 173 160 B1**
(45) Date of publication and mention of the grant of the patent: **22.06.2005**
(21) Application number: 00921553.4
(22) Date of filing: 31.03.2000
(51) Int. Cl.: A61K 31/10, C07C 317/10, C07C 321/20, C07C 317/44

(54) **(E)-STYRYL SULFONE ANTICANCER AGENTS**
(E)-STYRYLSULFONE ALS KREBSBESTREITENDE MITTEL
AGENTS ANTICANCER AU (E)-STYRYLSULFONE

(30) Priority: 02.04.1999 US 127683 P; 15.07.1999 US 143975 P
(43) Date of publication of application: 23.01.2002
(73) Proprietor: Temple University of the Commonwealth System of Higher Education, Philadelphia PA 19122 (US)
(72) Inventor: REDDY, E., Premkumar, Villanova, PA 19085 (US); REDDY, M., V., Ramana, Upper Darby, PA 19082 (US)
(74) Representative: Ouzman, Beverley Nicola Claire
(86) International application number: PCT/US2000/008565
(87) International publication number: WO 2000/059495

(56) References cited:
- EP-A- 1 223 923
- WO-A-00/57872
- WO-A-00/59494
- WO-A-99/18068
- US-A- 3 463 774
- DATABASE CA [Online] CHEMICAL ABSTRACTS SERVICE, COLUMBUS, OHIO, US; RIAD, Y. ET AL: "Mechanism of the reaction of sodium hydroxide and nitrobenzylsulfones" retrieved from STN Database accession no. 123:198103 XP002222278 & EGYPTIAN JOURNAL OF CHEMISTRY (1994), 37(2), 157-71 ,
- REDDY, D. BHASKAR ET AL: "Stereospecific synthesis of some new Z- and E-cyclopropyl benzyl sulfones and E,Z- and E,E-bis(cyclopropyl)sulfones by PTC method" PHOSPHORUS, SULFUR AND SILICON AND THE RELATED ELEMENTS (1994), 90(1-4), 1-10 , XP001070032
- REDDY, D. BHASKAR ET AL: "Phase transfer catalysis - a facile method for cyclopropanation of some isomeric styryl benzyl sulfones and bis(styryl)sulfones" ACH - MODELS IN CHEMISTRY (1994), 131(1), 83-92 , XP001061475
- REDDY, M. V. RAMANA ET AL: "A new route for the synthesis of styryl benzyl sulfones, precursors of 1-(benzylsulfonyl)-2-arylcyclopropanes" PHOSPHORUS, SULFUR AND SILICON AND THE RELATED ELEMENTS (1990), 53(1-4), 285-90 , XP001070034
- REDDY, D. BHASKAR ET AL: "Synthesis and cyclopropanation of (E)- and (Z)-styryl benzyl sulfones" SULFUR LETTERS (1991), 13(2), 83-90 , XP001062632
- REDDY, D. BHASKAR ET AL: "Preparation of styryl benzyl sulfones and 1,2-bis(styrylsulfonylmethyl)- 4,5-dimethylbenzenes" ORGANIC PREPARATIONS AND PROCEDURES INTERNATIONAL (1988), 20(3), 205-12 , XP001063393
- KAMIGATA, NOBUMASA ET AL: "Desulfonylation of arylmethanesulfonyl chlorides catalyzed by dichlorotris(triphenylphosphine)ruthenium( II)" PHOSPHORUS AND SULFUR AND THE RELATED ELEMENTS (1984), 20(2), 139-44 , XP001070033
- MAKOSZA, MIECZYSLAW ET AL: "Some reactions of the (chloromethyl)-trans-.beta.-styrylsulfone carbanion" LIEBIGS ANNALEN/RECUEIL (1997), (11), 2337-2340 , XP002222277
- BALIAH, V. ET AL: "Preparation and absorption spectra of some cis- and trans-.alpha..beta.- unsaturated sulfides and sulfones" INDIAN J. CHEM. (1971), 9(3), 220-5 , XP009001787
- DATABASE CAPLUS ON STN CHEMICAL ABSTRACTS (COLUMBUS, OHIO, USA) ACCESSION NO. 1997:304567 EVANS P.: 'The epoxy-Ramberg-Baecklund reaction: a new route to allylic alcohols' & TETRAHEDRON LETT. vol. 38, no. 17, 1997, pages 3055 - 3058
- DATABASE CAPLUS ON STN CHEMICAL ABSTRACTS (COLUMBUS, OHIO, USA) ACCESSION NO. 1989:74956 REDDY D.B.: 'Preparation of styryl benzyl sulfones and 1,2-bis(styrylsulfonylmethyl)-4, 5-dimethylbenzenes' & ORG. PREP. PROCED. INT. vol. 30, no. 3, 1988, pages 205 - 212
- DATABASE CAPLUS ON STN CHEMICAL ABSTRACTS (COLUMBUS, OHIO, USA) ACCESSION NO. 1984:423063 REDDY M.V.R.: 'Synthesis of alpha beta-unsaturated sulfones' & ACTA CHIM. HUNG. vol. 115, no. 3, March 1984, pages 269 - 271
- DATABASE CAPLUS ON STN CHEMICAL ABSTRACTS (COLUMBUS, OHIO, USA) ACCESSION NO. 1996:651386 RIAD Y.: 'Kinetics and mechanism of the catalytic reduction of (((4-nitrophenyl)-methyl)sulfonyl) acetic acid in alkaline dioxane-water media' & EGYPT. J. vol. 39, no. 4, 1996, pages 353 - 364
- DATABASE CAPLUS ON STN CHEMICAL ABSTRACTS (COLUMBUS, OHIO, USA) ACCESSION NO. 1994:210378 CHENG H.M.: 'Metabolism of 14C-ring-labeled benthiocarb in mice and rats' & J. CHIN. BIOCHEM. SOC. vol. 22, no. 1, 1993, pages 27 - 35
- DATABASE CAPLUS ON STN CHEMICAL ABSTRACTS (COLUMBUS, OHIO, USA) ACCESSION NO. 1985:541088 JANCZEWSKI M.: 'Effect of molecular structure on optical properties of sulfoxide systems. m-Bromobenzylsulfinylacetic acids and some of their derivatives' & POL. J. CHEM. vol. 58, no. 1-2-3, 1984, pages 103 - 116
- DATABASE CAPLUS ON STN CHEMICAL ABSTRACTS (COLUMBUS, OHIO, USA) ACCESSION NO. 1994:656180 LI C.: 'Synthesis and pharmacological evaluation of vinyl sulfone based anticancer agents' & BIOORG. MED. CHEM. LETT. vol. 4, no. 13, 1994, pages 1585 - 1590

## Description

### Cross-Reference to Related Applications

The benefit of the filing date of U.S. provisional patent applications Ser. Nos. 60/127,683, filed April 2 1999, and 60/143,975 filed July 15, 1999, is hereby claimed pursuant to 35 U.S.C. 119(e).

### Field of the Invention

The invention relates to compositions and methods for the treatment of cancer.

### Background of the Invention

Extracellular signals received at transmembrane receptors are relayed into the cells by the signal transduction pathways (Pelech *et al*., *Science* **257**:1335 (1992)) which have been implicated in a wide array of physiological processes such as induction of cell proliferation, differentiation or apoptosis (Davis *et al*., *J. Biol. Chem.* **268**:14553 (1993)). The Mitogen Activated Protein Kinase (MARK) cascade is a major signaling system by which cells transduce extracellular cues into intracellular responses (Nishida *et al*., *Trends Biochem. Sci.* **18**:128 (1993); Blumer *et al*., *Trends Biochem. Sci.* **19**:236 (1994)). Many steps of this cascade are conserved, and homologous for MAP kinases have been discovered in different species.

In mammalian cells, the Extracellular-Signal-Regulated Kinases (ERKs), ERK-1 and ERK-2 are the archetypal and best-studied members of the MAPK family, which all have the unique feature of being activated by phosphorylation on threonine and tyrosine residues by an upstream dual specificity kinase (Posada *et al*., *Science* **255**:212 (1992); Biggs III *et al*., *Proc. Natl. Acad. Sci. USA* **89**:6295 (1992); Garner *et al*., *Genes Dev.* **6**:1280 (1992)).

Recent studies have identified an additional subgroup of MAPKs, known as c-Jun NH2-terminal kinases 1 and 2 (JNK-1 and JNK-2), that have different substrate specificities and are regulated by different stimuli (Hibi *et al*., *Genes Dev.* **7**:2135 (1993)). JNKs are members of the class of stress-activated protein kinases (SPKs). JNKs have been shown to be activated by treatment of cells with UV radiation, pro-inflammatory cytokines and environmental stress (Derijard *et al*., *Cell* 1025 (1994)). The activated JNK binds to the amino terminus of the c-Jun protein and increases the protein's transcriptional activity by phosphorylating it at ser63 and ser73 (Adler *et al., Proc. Natl. Acad. Sci. USA* **89**:5341 (1992); Kwok *et al*., *Nature* **370**:223 (1994)).

Analysis of the deduced primary sequence of the JNKs indicates that they are distantly related to ERKs (Davis, *Trends Biochem. Sci.* **19**:470 (1994)). Both ERKs and JNKs are phosphorylated on Tyr and Thr in response to external stimuli resulting in their activation (Davis, *Trends Biochem. Sci*. **19**:470 (1994)). The phosphorylation (Thr and Tyr) sites, which play a critical role in their activation are conserved between ERKs and JNKs (Davis, *Trends Biochem. Sci.* **19**:470 (1994)). However, these sites of phosphorylation are located within distinct dual phosphorylation motifs: Thr-Pro-Tyr (JNK) and Thr-Glu-Tyr (ERK). Phosphorylation of MAPKs and JNKs by an external signal often involves the activation of protein tyrosine kinases (PTKs) (Gille *et al*., *Nature* **358**:414 (1992)), which constitute a large family of proteins encompassing several growth factor receptors and other signal transducing molecules.

Protein tyrosine kinases are enzymes which catalyze a well defined chemical reaction: the phosphorylation of a tyrosine residue (Hunter *et al*., *Annu Rev Biochem* **54**:897 (1985)). Receptor tyrosine kinases in particular are attractive targets for drug design since blockers for the substrate domain of these kinases is likely to yield an effective and selective antiproliferative agent. The potential use of protein tyrosine kinase blockers as antiproliferative agents was recognized as early as 1981, when quercetin was suggested as a PTK blocker (Graziani *et al*., *Eur. J. Biochem*. **135**:583-589 (1983)).

The best understood MAPK pathway involves extracellular signal-regulated kinases which constitute the Ras/Raf/MEK/ERK kinase cascade (Boudewijn *et al*., *Trends Biochem. Sci.* 20, 18 (1995)). Once this pathway is activated by different stimuli, MAPK phosphorylates a variety of proteins including several transcription factors which translocate into the nucleus and activate gene transcription. Negative regulation of this pathway could arrest the cascade of these events.

What are needed are new anticancer chemotherapeutic agents which target receptor tyrosine kinases and which arrest the Ras/Raf/MEK/ERK kinase cascade. Oncoproteins in general, and signal transducing proteins in particular, are likely to be more selective targets for chemotherapy because they represent a subclass of proteins whose activities are essential for cell proliferation, and because their activities are greatly amplified in proliferative diseases.

What is also needed are new anticancer therapeutics which are highly selective in the killing of tumor cells, but not normal cells.

### Summary of the Invention

It is an object of the invention to provide compounds, compositions and methods for the treatment of cancer and other proliferative diseases. The biologically active compounds are in the form of (E)-styryl benzylsulfones.

It is an object of the invention to provide compounds which are highly selective in killing tumor cells but not normal cells.

It is a further object of the invention to provide novel polymers prepared by polymerisation of (E)-styryl benzylsulfones.

It is a further object of the invention to provide intermediates useful for the preparation of compounds having anticancer activity. The intermediates comprise (E)-styryl benzylsulfonyl acetic acids.

According to one embodiment of the invention, novel compounds are provided according to formula I: 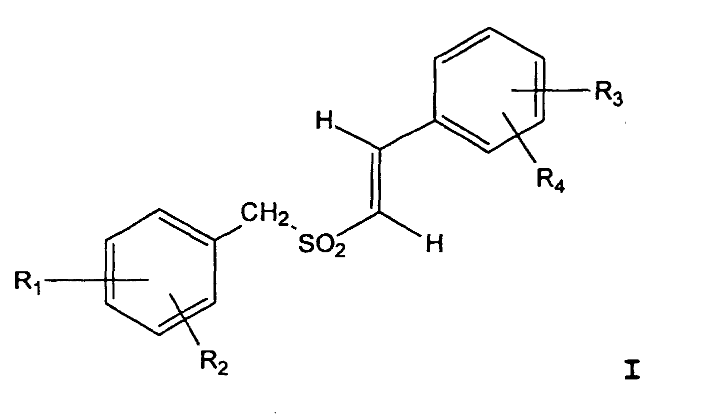 wherein:
R₁ and R₂ are independently selected from the group consisting of hydrogen, fluoro, chloro, bromo, C1-C6 alkyl, C1-C6 alkoxy, nitro, cyano and trifluoromethyl;
R₃ and R₄ are independently selected from the group consisting of hydrogen, fluoro, chloro, bromo, nitro, cyano and trifluoromethyl;
at least one of R₁ and R₂ is other than hydrogen; and
at least one of R₃ and R₄ is other than hydrogen;
   with the proviso that
   (a) when R₁ and R₃ are hydrogen and R₂ is 4-chloro, then R₄ may not be 4-chloro, 4-fluoro, 4-bromo, or 4-nitro;
   (b) when R₁ and R₃ are hydrogen and R₂ is 4-fluoro or 4-bromo, then R₄ may not be 4-fluoro, 4-bromo, or 4-chloro;
   (c) when R₁ and R₃ are hydrogen and R₂ is 4-nitro, then R₄ may not be 4-chloro, 4-nitro, 4-bromo, or 4-fluoro;
   (d) when R₁ and R₃ are hydrogen and R₂ is 4-methyl, then R₄ may not be 4-chloro, 4-bromo, 4-fluoro, or 2-chloro;
   (e) when R₁ is hydrogen, then R₂, R₃ and R₄ may not all be -fluoro;
   (f) when R₁ is hydrogen and R₃ is 2-fluoro, then R₂ and R₄ may not both be selected from the group consisting of 4-chloro, 4-bromo and 4-fluoro; and
   (g) the compound is not (E)-2-chloro-4-fluorostyryl-4-fluorobenzyl sulfone, (E)-2-chloro-4-fluorostyryl-4-chlorobenzyl sulfone, (E)-2-chloro-4-fluorostyryl-4-bromobenzyl sulfone, (E)-2,4-dichlorostyryl-4-fluorobenzyl sulfone, (E)-2,4-dichlorostyryl-4-chlorobenzyl sulfone, (E)-2,4-dichlorostyryl-4-bromobenzyl sulfone, (E)-2-chloro-4-bromostyryl-4-fluorobenzyl sulfone, (E)-2-chloro-4-bromostyryl-4-chlorobenzyl sulfone, or (E)-2-chloro-4-bromostyryl-4-bromobenzyl sulfone.

According to a preferred embodiment of the invention, novel compounds are provided according to formula I wherein R₁, R₂, R₃ and R₄ are independently selected from the group consisting of hydrogen, chloro, fluoro, bromo, nitro, cyano and trifluoromethyl. According to a more preferred embodiment, R₁, R₂, R₃ and R₄ are independently selected from the group consisting of hydrogen, chloro, fluoro and bromo; most preferably hydrogen, chloro and fluoro.

In a further preferred embodiment, novel compounds are provided according to formula I wherein (1) at least one of R₁ and R₂ is located at the 2-, 3- and/or 4- position of the phenyl ring to which it is attached, and is preferably selected from chloro and fluoro, most preferably chloro; and/or (2) wherein at least one of R₃ and R₄ is located at the 2- and/or 4- position of the phenyl ring to which it is attached, and is preferably selected from chloro and fluoro. In other preferred embodiments, (i) R₂ is 4-halogen or 4-cyano, and R₄ is 4-nitro; or (ii) R₂ is 4-C1-C6 alkoxy, and R₄ is 4-nitro or 4-halogen. R₁ and R₃ are preferably hydrogen in these embodiments.

In another embodiment of the invention, a pharmaceutical composition is provided comprising a pharmaceutically acceptable carrier and one or more compounds of formula I as described above. According to a further aspect of the present invention, compounds for use in medicine are provided according to formula III: 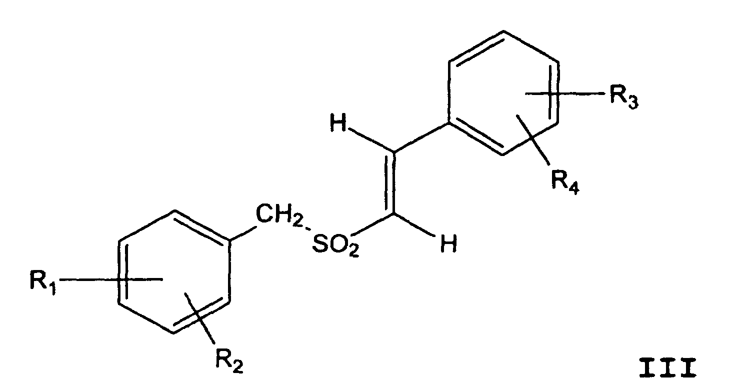 wherein:
R₁ and R₂ are independently selected from the group consisting of hydrogen, fluoro, chloro, bromo, C1-C6 alkyl, C1-C6 alkoxy, nitro, cyano and trifluoromethyl;
at least one of R₁ and R₂ is other than hydrogen; and at least one of R₃ and R₄ is other than hydrogen;
   with the proviso that
   (a) when R₁ and R₃ are hydrogen and R₂ is 4-bromo or 4-chloro, R₄ may not be 4-chloro, 4-fluoro, or 4-bromo;
   (b) when R₁ and R₃ are hydrogen and R₂ is 4-fluoro, R₄ may not be 4-fluoro or 4-bromo;
   (c) when R₁ is hydrogen and R₄ is 2-fluoro, then R₂ and R₃ may not be 4-fluoro;
   (d) when R₁ is hydrogen and R₃ is 4-hydrogen, 4-chloro, or 4-bromo, and R₄ is 2-hydrogen, 2-chloro or 2-fluoro, then R₂ may not be 4-hydrogen, 4-chloro, 4-fluoro, or 4-bromo; and
   (e) the compound is not (*E*)-2-chloro-4-fluorostyryl-4-fluorobenzyl sulfone, (*E*)-2-chloro-4-fluorostyryl-4-chlorobenzyl sulfone, (*E*)-2-chloro-4-fluorostyryl-4-bromobenzyl sulfone, (*E*)-2,4-difluorostyryl-4-chlorobenzyl sulfone, or (*E*)-2,4-difluorostyryl-4-bromobenzyl sulfone.

According to a preferred embodiment, at least one of R₁ and R₂ in formula III is located at the 2- or 4-position of the phenyl ring to which it is attached; and at least one of R₃ and R₄ is located at the 2- or 4-position of the phenyl ring to which it is attached. According to a further preferred embodiment, R₂ and R₄ in formula III are hydrogen, and R₁ and R₃ are located at the 4-position of the phenyl rings to which they are attached. According to a further preferred embodiment, one of R₁ or R₃ is selected from the group consisting of chloro, fluoro, bromo and nitro.

A pharmaceutical composition is also provided comprising a pharmaceutically acceptable carrier and one or more compounds of formula III above, wherein R₁, R₂, R₃ and R₄ are defined as above for formula III.

Where R₁ or R₂ is an alkyl or alkoxy group in any compound of formulae I or III, the carbon chain may be branched or straight, with straight being preferred. Preferably, the alkyl and alkoxy groups comprise C1-C3 alkyl and C1-C4 alkoxy, most preferably methyl and methoxy.

According to another embodiment of the invention, the use of a compound according to formula I or III is provided in the preparation of a medicament for treatment of a proliferative disorder, particularly cancer.

In another embodiment of the invention, the use of a compound according to formula I or III is provided in the preparation of a medicament for inducing apoptosis of tumor cells in an individual afflicted with cancer.

The present invention also provides a series of substituted benzylsulfonyl acetic acid compounds having structural formula V, below: 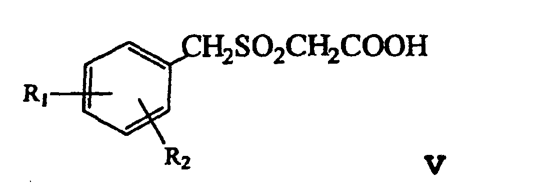 wherein:
R₁ and R₂ are both halogen; or
R₁ is hydrogen and R₂ is CF₃;
or a salt thereof, provided that the compound is other than 2,4-dichlorobenzylsulfonylacetic acid.

The substituted benzylsulfonyl acetic acid compounds are useful as intermediates in the synthesis of novel (E)-styrylbenzylsulfone compounds of formula I, according to Method A, below.

### Detailed Description of the Invention

According to the present invention, certain (E)-styrylbenzylsulfone derivatives selectively kill various tumor cell types without killing normal cells. Without wishing to be bound by any theory, it is believed that the compounds affect the MAPK signal transduction pathway, thereby affecting tumor cell growth and viability. This cell growth inhibition is associated with regulation of the ERK and JNK types of MAPK. Without wishing to be bound by any theory, the styryl sulfones of the present invention may block the phosphorylating capacity of ERK-2.

The compounds of the invention have been shown to inhibit the proliferation of tumor cells by inducing cell death. The compounds are believed effective against a broad range of tumor types, including but not limited to the following: breast, prostate, ovarian, lung, colorectal, brain (i.e, glioma) and renal. The compounds are also believed effective against leukemic cells. The compounds do not kill normal cells in concentrations at which tumor cells are killed.

The compounds are also useful in the treatment of non-cancer proliferative disorders, including but not limited to the following: hemangiomatosis in new born, secondary progressive multiple sclerosis, chronic progressive myelodegenerative disease, neurofibromatosis, ganlioneuromatosis, keloid formation, Pagets Disease of the bone, fibrocystic disease of the breast, Peronies and Duputren's fibrosis, restenosis and cirrhosis.

Treatment of this broad range of tumor cells with the styryl benzylsulfone compounds of the invention leads to inhibition of cell proliferation and induction of apoptotic cell death. In breast tumors, the effect is observed for estrogen receptor (ER) positive as well as estrogen receptor negative cells.

Tumor cells treated with the compounds of the invention accumulate in the G2/M phase of the cell cycle. As the cells exit the G2/M phase, they appear to undergo apoptosis. Treatment of normal cells with the styryl sulfones does not result in apoptosis.

### Synthesis of (E)- Styryl Benzylsulfones

The styryl benzylsulfones are characterized by cis-trans isomerism resulting from the presence of one or more double bonds. The compounds are named according to the Cahn-Ingold-Prelog system, the IUPAC 1974 Recommendations, Section E: Stereochemistry, in *Nomenclature of Organic Chemistry,* John Wiley & Sons, Inc., New York, NY, 4^{th} ed., 1992, p. 127-138. Stearic relations around a double bond are designated as "Z" or "E".

(*E*)-styryl benzylsulfones are prepared by Knoevenagel condensation of aromatic aldehydes with benzylsulfonyl acetic acids. The procedure is described by Reddy *et al*., *Acta. Chim. Hung.* **115**:269 (1984); Reddy *et al*., *Sulfur Letters* **13**:83 (1991); Reddy *et al*., *Synthesis 322* (1984); and Reddy *et al*., *Sulfur Letters* **7**:43 (1987).

The (*E*)-styryl benzylsulfones can be prepared according to either of the following Methods A and B:

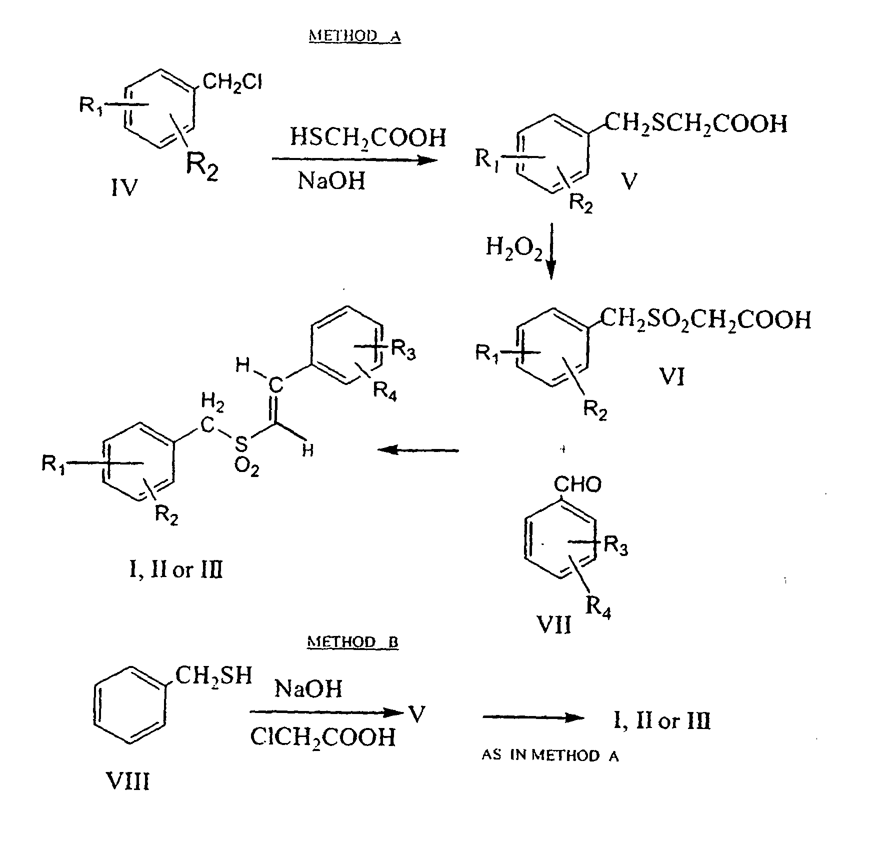

### METHOD A

A benzyl thioacetic acid V formed by the reaction of sodium thioglycollate and a benzyl chloride IV. The benzyl thioacetic acid V is oxidized with 30% hydrogen peroxide to give a corresponding benzylsulfonyl acetic acid VI. Condensation of VI with an aromatic aldehyde VII via a Knoevenagel reaction in the presence of benzylamine and glacial acetic acid yields the (E)-styryl benzylsulfone I, II or III.

### METHOD B

A benzylthioacetic acid V is formed by the reaction of the appropriate sodium benzylthiolate VIII with chloroacetic acid. Oxidation of V to the corresponding benzylsulfonyl acetic acid VI and subsequent Knoevenagel condensation with aldehyde VII is carried out as in Method A.

Substituted benzylsulfonyl acetic acid compounds Va, Vb, Vc, and Vd according to formula V were prepared by reacting the corresponding benzyl chloride with thioglycollic acid under basic conditions (Method A). These compounds are novel intermediates.

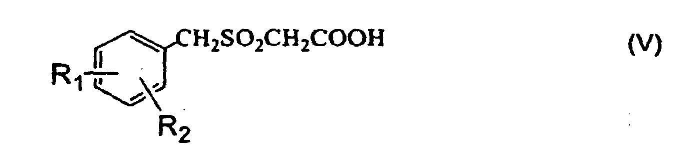

| **No.** | **Compound** | **R**_{**1**} | **R**_{**2**} | **M.P. (°C)** |
|---|---|---|---|---|
| Technical information Va | 4-nitrobenzylsulfonyl acetic acid | H | 4-NO₂ | 165-166 |
| Vb | 4-trifluoromethylbenzylsulfonyl acetic acid | H | 4-CF₃ | 164-165 |
| Technical information Vc | 2,4-dichlorobenzylsulfonyl acetic acid | 2-Cl | 4-Cl | 165-166 |
| Vd | 3,4-dichlorobenzylsulfonyl acetic acid | 3-Cl | 4-Cl | 132-134 |

The (E)-styryl benzylsulfones may be derivatized with a chemical group to permit conjugation to a carrier molecule, for the purpose of raising antibodies to the styryl sulfones. Suitable derivatizing chemistries are well-known to those skilled In the art. Preferably, the derivative comprises a carboxylic acid derivative. The carrier may comprise any molecule sufficiently large to be capable of generating an Immune response in an appropriate host animal. One such preferred carrier is keyhole limpet haemocyanin (KLH).

### Therapeutic Administration

The (E)-styryl benzylsulfones of the invention may be administered in the form of a pharmaceutical composition, in combination with a pharmaceutically acceptable carrier. The active ingredient in such formulations may comprise from 0.1 to 99.99 weight percent. By "pharmaceutically acceptable carrier" is meant any carrier, diluent or excipient which is compatible with the other ingredients of the formulation and to deleterious to the recipient.

The compounds of the invention may be administered to individuals (mammals, including animals and humans) afflicted with cancer. The compounds are also useful in the treatment of non-cancer proliferative disorders, that is, proliferative disorders which are characterized by benign indications. Such disorders may also be known as "cytoproliferative" or "hyperproliferative" in that cells are made by the body at an atypically elevated rate. Such disorders include, but are not limited to, the following: hemangiomatosis in new born, secondary progressive multiple sclerosis, chronic progressive myelodegenerative disease, neurofibromatosis, ganglioneuromatosis, keloid formation, Pagets Disease of the bone, fibrocystic disease of the breast, Peronies and Duputren's fibrosis, restenosis and cirrhosis.

The compounds may be administered by any route, including oral and parenteral administration. Parenteral administration includes, for example, intravenous, intramuscular, intraarterial, intraperitoneal, intranasal, rectal, intravaginal, topical or subcutaneous administration. The active agent is preferably administered with a pharmaceutically acceptable carrier selected on the basis of the selected route of administration and standard pharmaceutical practice.

The active agent may be formulated into dosage forms according to standard practices in the field of pharmaceutical preparations. See Gennaro Alphonso, ed., *Remington's Pharmaceutical Sciences,* 18th Ed., (1990) Mack Publishing Co., Easton, PA. Suitable dosage forms may comprise, for example, tablets, capsules, solutions, parenteral solutions, troches, suppositories, or suspensions.

For parenteral administration, the active agent may be mixed with a suitable carrier or diluent such as water, an oil (particularly a vegetable oil), ethanol, saline solution, aqueous dextrose (glucose) and related sugar solutions, glycerol, or a glycol such as propylene glycol or polyethylene glycol. Solutions for parenteral administration preferably contain a water soluble salt of the active agent. Stabilizing agents, antioxidizing agents and preservatives may also be added. Suitable antioxidizing agents include sulfite, ascorbic acid, citric acid and its salts, and sodium EDTA. Suitable preservatives include benzalkonium chloride, methyl- or propyl-paraben, and chlorbutanol. The composition for parenteral administration may take the form of an aqueous or nonaqueous solution, dispersion, suspension or emulsion.

For oral administration, the active agent may be combined with one or more solid inactive ingredients for the preparation of tablets, capsules, pills, powders, granules or other suitable oral dosage forms. For example, the active agent may be combined with at least one excipient such as fillers, binders, humectants, disintegrating agents, solution retarders, absorption accelerators, wetting agents absorbents or lubricating agents. According to one tablet embodiment, the active agent may be combined with carboxymethylcellulose calcium, magnesium stearate, mannitol and starch, and then formed into tablets by conventional tableting methods.

The specific dose of compound according to the invention to obtain therapeutic benefit will, of course, be determined by the particular circumstances of the individual patient including, the size, weight, age and sex of the patient, the nature and stage of the disease, the aggressiveness of the disease, and the route of administration. For example, a daily dosage of from 0.05 to 50 mg/kg/day may be utilized. Higher or lower doses are also contemplated.

### Examples

### General Procedure for Synthesis (E)-Styryl Benzylsulfones

**Part A.** To a solution of (8g, 0.2 mol) sodium hydroxide in methanol (200 ml), thioglycollic acid (0.1 mol) is added slowly and the precipitate formed is dissolved by stirring the contents of the flask. Then an appropriately substituted or unsubstituted benzyl chloride (0.1 mol) is added stepwise and the reaction mixture is refluxed for 2-3 hours. The cooled contents are poured onto crushed ice and neutralized with dilute hydrochloric acid (200 ml). The resulting corresponding benzylthioacetic acid (0.1 mol) is subjected to oxidation with 30% hydrogen peroxide (0.12 mol) in glacial acetic acid (125 ml) by refluxing for 1 hour. The contents are cooled and poured onto crushed ice. The separated solid is recrystalized from hot water to give the corresponding pure benzylsulfonylacetic acid.

**Part B.** A mixture of the benzylsulfonyl acetic acid (10 mmol), an appropriately substituted or unsubstituted aromatic aldehyde (10 mmol), and benzylamine (200 µl) in glacial acetic acid (12 ml) is refluxed for 2-3 hours. The contents are cooled and treated with cold ether (50 ml). Any product precipitated out is separated by filtration. The filtrate is diluted with more ether and washed successively with a saturated solution of sodium bicarbonate (20 ml), sodium bisulfite (20 ml), dilute hydrochloric acid (20 ml) and finally with water (35 ml). Evaporation of the dried ethereal layer yields styryl benzylsulfones as a solid material.

In each of the following examples, the substituted benzylsulfonyl acetic acid was made according to Part A of the General Procedure. All the styryl benzylsulfone compounds were recrystalized from 2-propanol and the purity was checked by thin layer chromatography.

### Example 1

### (E)-4-Fluorostyryl-4-trifluoromethylbenzylsulfone

A solution of 4-trifluorobenzylsulfonylacetic acid (10 mmol) and 4-fluorobenzaldehyde (10mmol) was subjected to the General Procedure, Part B. The title compound melting point 166-168°C, was obtained in 82% yield.

### Example 2

### (E)-4-Chlorostyryl-4-trifluoromethylbenzylsulfone

A solution of 4-trifluoromethylbenzylsulfonylacetic acid (10 mmol) and 4-chlorobenzaldehyde (10 mmol) was subjected to the General Procedure, Part B. The title compound, melting point 164-168°C, was obtained in 88% yield.

### Example 3

### (E)-4-Bromostyryl-4-trifluoromethylbenzylsulfone

A solution of 4-trifluoromethylbenzylsulfonylacetic acid (10 mmol) and 4-bromobenzaldehyde (10 mmol) was subjected to the General Procedure, Part B. The title compound, melting point 181-183°C, was obtained in 85% yield.

### Example 4

### (E)-4-Fluorostyryl-2,4-dichlorobenzylsulfone

A solution of 2,4-dichlorobenzylsulfonyl acid (10 mmol) and 4-fluorobenzaldehyde (10 mmol) was subjected to the General Procedure, Part B. The title compound, melting point 146-148°C, was obtained in 78% yield.

### Example 5

### (E)-4-Chlorostyryl-2,4-dichlorobenzylsulfone

A solution of 2,4-dichlorobenzylsulfonylacetic acid (10 mmol) and 4-chlorobenzaldehyde (10 mmol) was subjected to the General Procedure, Part B. The title compound, melting point 148-149°C, was obtained in 84% yield.

### Example 6

### (E)-4-Fluorostyryl-3,4-dichlorobenzylsulfone

A solution of 3,4-dichlorobenzylsulfonylacetic acid (10 mmol) and 4-fluorobenzaldehyde (10 mmol) was subjected to the General Procedure, Part B. The title compound, melting point 120-122°C, was obtained in 82% yield.

### Example 7

### (E)-4-Chlorostyryl-3,4-dichlorobenzylsulfone

A solution of 3,4-dichlorobenzylsulfonylacetic acid (10 mmol) and 4-chlorobenzaldehyde (10 mmol) was subjected to the General Procedure, Part B. The title compound, melting point 149-151°C, was obtained in 86% yield.

### Example 8

### (E)-4-Bromostyryl-3,4-dichlorobenzylsulfone

A solution of 3,4-dichlorobenzylsulfonylacetic acid (10 mmol) and 4-bromobenzaldehyde (10 mmol) was subjected to the General Procedure, Part B. The title compound, melting point 154-155°C, was obtained in 84% yield.

### Example 9

### (E)-4-Fluorostyryl-4-nitrobenzylsulfone

A solution of 4-nitrobenzylsulfonylacetic acid (10 mmol) and 4-fluorobenzaldehyde (10 mmol) was subjected to the General Procedure, Part B. The title compound, melting point 160-161°C, was obtained in 76% yield.

### Example 10

### (E)-4-Fluorostyryl-4-cyanobenzylsulfone

A solution of 4-cyanobenzysulfonylacetic acid (10 mmol) and 4-fluorobenzaldehyde (10 mmol) was subjected to the General Procedure Part B. The title compound, melting point 150-151°C, was obtained in 82% yield.

### Example 11

### (E)-4-Chlorostyryl-4-cyanobenzylsulfone

A solution of 4-cyanobenzylsulfonyl acetic acid (10 mmol) and 4-chlorobenzaldehyde (10 mmol) was subjected to the General Procedure, Part B. The title compound, melting point 173-177°C, was obtained in 86% yield.

### Example 12

### (E)-4-Bromostyryl-4-cyanobenzylsulfone

A solution of 4-cyanobenzylsulfonyl acetic acid (10 mmol) and 4-bromobenzaldehyde (10 mmol) was subjected to the General Procedure, Part B. The title compound, melting point 183-184°C, was obtained in 77% yield.

### Example 13

### (E)-3,4-Difluorostyryl-4-chlorobenzylsulfone

A solution of 4-chlorobenzylsulfonyl acetic acid (10 mmol) and 3,4 difluorobenzaldehyde was subjected to the General Procedure, Part B. The title compound, melting point 204-205°C, was obtained in 73% yield.

### Example 14

### (E)-3-Chloro-4-fluorostyryl-4-chlorobenzylsulfone

A solution of 4-chlorobenzylsulfonylacetic acid (10 mmol) and 3-chloro-4-fluorobenzaldehyde was subjected to the General Procedure, Part B. The title compound, melting point 181-183°C, was obtained in 78% yield.

### Example 15

### (E)-2-Chloro-4-fluorostyryl-4-chlorobenzylsulfone

A solution of 4-chlorobenzylsulfonylacetic acid (10 mmol) and 2-chloro-4-fluorobenzaldehyde was subjected to the General Procedure, Part B. The title compound, melting point 149-150°C, was obtained in 68% yield.

### Example 16 - Technical information

### (E)-2,4-Dichlorostyryl-4-chlorobenzylsulfone

A solution of 4-chlorobenzyls ulfonylacetic acid (10 mmol) and 2,4-dichlorobenzaldehyde was subjected to the General Procedure, Part B. The title compound, melting point 164-165°C, was obtained in 78% yield.

### Example 17

### (E)-3,4-Dichlorostyryl-4-chlorobenzylsulfone

A solution of 4-chlorobenzylsulfonyl acetic acid (10 mmol) and 3,4-dichlorobenzaldehyde (10 mmol) was subjected to the General procedure, Part B. The title compound, melting point 170-171 °C, was obtained in 73% yield.

### Example 18

### (E)-2,3-Dichlorostyryl-4-chlorobenzylsulfone

A solution of 4-chlorobenzylsulfonyl acetic acid (10 mmol) and 2,3-dichlorobenzaldehyde (10 mmol) was subjected to the General Procedure, part B. The title compound, melting point 170-171°C, was obtained in 72% yield.

### Example 19 - Technical information

### (E)-4-Fluorostyryl-4-iodobenzylsulfone

A solution of 4-iodobenzylsulfonyl acetic acid (10 mmol) and 4-fluorobenzaldehyde (10 mmol) was subjected to the General Procedure, part B. The title compound, melting point 171-173°C, was obtained in 98% yield. (1HNMR, CDCl3) d 4.27(s, CH2), 6.60 (d, = CH, J = 15.7 Hz), 7.18-7.80 (m, 9H, Aroma + = CH).

### Example 20 - Technical information

### (E)-4-Iodostyryl-4-fluorobenzylsulfone

A solution of 4-fluorobenzylsulfonyl acetic acid (10 mmol) and 4-iodobenzaldehyde (10 mmol)was subjected to the General Procedure, part B. The title compound, melting point 168-170°C, was obtained in 58% yield.

### Example 21 -Technical information

### (E)-4-Iodostyryl-4-chlorobenzylsulfone

A solution of 4-chlorobenzylsulfonyl acetic acid (10 mmol) and 4-iodobenzaldehyde (10 mmol) was subjected to the General Procedure, part B. The title compound, melting point 181-182°C, was obtained in 70% yield. (1HNMR, CDCl3) d 4.27(s, CH2), 6.60 (d, = CH, J = 15.7 Hz), 7.18-7.80 (m, 9H, Aroma + = CH).

### Example 22 - Technical information

### (E)-4-Iodostyryl-4-bromobenzylsulfone

A solution of 4-bromobenzylsulfonyl acetic acid (10 mmol) and 4-iodobenzaldehyde (10 mmol)was subjected to the General Procedure, part B. The title compound, melting point 201-203°C, was obtained in 71% yield.

### Example 23 -Technical information

### (E)-4-Chlorostyryl-4-iodobenzylsulfone

A solution of 4-iodobenzylsulfonyl acetic acid (10 mmol) and 4-chlorobenzaldehyde (10 mmol) was subjected to the General Procedure, part B. The title compound, melting point 200-202 °C, was obtained in 86% yield. (1HNMR, CDCl3) d 4.27(s, CH2), 6.60 (d, = CH, J = 15.7 Hz), 7.18-7.80 (m, 9H, Aroma + = CH).

### Example 24 - Technical information

### (E)-4-Bromostyryl-4-iodobenzylsulfone

A solution of 4-iodobenzylsulfonyl acetic acid (10 mmol) and 4-bromobenzaldehyde (10 mmol) was subjected to the General Procedure, part B. The title compound, melting point 217-219 °C, was obtained in 88% yield.

### Example 25 -Technical information

### (E)-2-Nitrostyryl-4-iodobenzylsulfone

A solution of 4-iodobenzylsulfonyl acetic acid (10 mmol) and 2-nitrobenzaldehyde (10 mmol) was subjected to the General Procedure, part B. The title compound, melting point 227-229°C, was obtained in 62% yield.

### Example 26 -Technical information

### (E)-4-Nitrostyryl-4-iodobenzylsulfone

A solution of 4-iodobenzylsulfonyl acetic acid (10 mmol) and 4-nitrobenzaldehyde (10 mmol) was subjected to the General Procedure, part B. The title compound, melting point 227-228°C, was obtained in 62% yield.

### Example 27 - Technical information

### (E)-4-Iodostyryl-4-methoxybenzylsulfone

A solution of 4-methoxybenzylsulfonyl acetic acid (10 mmol) and 4-iodobenzaldehyde (10 mmol) was subjected to the General Procedure, part B. The title compound, melting point 201-203°C, was obtained in 56% yield.

### Example 28 - Technical information

### (E)-4-Iodostyryl-2,4-dichlorobenzylsulfone

A solution of 2,4-dichlorobenzylsulfonyl acetic acid (10 mmol) and 4-iodobenzaldehyde (10 mmol) was subjected to the General Procedure, part B. The title compound, melting point 181-182°C, was obtained in 60% yield.

The following additional compounds In Table 1 were prepared according to the same synthetic methods (M.P. = melting point):

**Table 1**

| **Ex.** | **M.P. (°C)** | **Yield (%)** | **Compound** |
|---|---|---|---|
| 29 | 134-136 | 55 | (E)-2-nitrostyryl-4-fluorobenzylsulfone |
| 30 | 170-173 | 64 | (E)-3-nitrostyryl-4-fluorobenzylsulfone |
| 31 | 151-152 | 61 | (E)-4-nitrostyryl-4-fluorobenzylsulfone |
| 32 | 96-98 | 54 | (E)-2-trifluoromethylstyryl-4-fluorobenzylsulfone |
| 33 | 117-119 | 55 | (E)-3-trifluoromethylstyryl-4-fluorobenzylsulfone |
| 34 | 125-128 | 73 | (E)-4-trifluoromethylstyryl-4-fluorobenzylsulfone |
| 35 | 108-112 | 52 | (E)-2-trifluoromethy-4-fluorostyryl-4-fluorobenzylsulfone |
| 36 | 128-132 | 58 | (E)-2-nitrostyryl-4-chlorobenzylsulfone |
| 37 | 156-157 | 60 | (E)-3-nitrostyryl-4-chlorobenzylsulfone |
| 38 | 189-191 | 61 | (E)-4-nitrostyryl-4-chlorobenzylsulfone |
| 39 | 100-101 | 55 | (E)-2-trifluoromethylstyryl-4-chlorobenzylsulfone |
| 40 | 155-157 | 58 | (E)-3-trifluoromethylstyryl-4-chlorobenzylsulfone |
| 41 | 164-166 | 59 | (E)-4-trifluoromethylstyryl-4-chlorobenzylsulfone |
| 42 | 115-117 | 63 | (E)-2-trifluoromethyl-4-fluorostyryl-4-chlorobenzylsulfone |
| 43 | 169-171 | 63 | (E)-3-methyl-4-fluorostyryl-4-chlorobenzylsulfone |
| 44 | 136-138 | 57 | (E)-2-nitrostyryl-2,4-dichlorobenzylsulfone |
| 45 | 136-138 | 57 | (E)-2-trifluoromethyl-4-fluorostyryl-2,4-dichlorobenzylsulfone |
| 46 | 131-132 | 63 | (E)-2-nitrostyryl-4-bromobenzylsulfone |
| 47 | 168-170 | 56 | (E)-3-nitrostyryl-4-bromobenzylsulfone |
| 48 | 205-207 | 67 | (E)-4-nitrostyryl-4-bromobenzylsulfone |
| 49 | 102-104 | 57 | (E)-2-trifluoromethylstyryl-4-bromobenzylsulfone |
| 50 | 160-161 | 55 | (E)-3-trifluoromethylstyryl-4-fluorobenzylsulfone |
| 51 | 174-175 | 62 | (E)-4-trifluoromethylstyryl-4-bromoyanobenzylsulfone |
| 52 | 167-168 | 63 | (E)-2-nitrostyryl-4-cyanobenzylsulfone |
| 53 | 192-193 | 62 | (E)-3-nitrostyryl-4-cyanobenzylsulfone |
| 54 | 219-220 | 66 | (E)-4-nitrostyryl-4-cyanobenzylsulfone |
| 55 | 182-184 | 70 | (E)-4-fluorostyryl-4-methylbenzylsulfone |
| 56 | 191-192 | 70 | (E)-4-bromostyryl-4-methylbenzylsulfone |
| 57 | 128-130 | 51 | (E)-2-nitrostyryl-4-methylbenzylsulfone |
| 58 | 201-203 | 56 | (E)-3-nitrostyryl-4-methylbenzylsulfone |
| 59 | 194-195 | 57 | (E)-4-nitrostyryl-4-methylbenzylsulfone |
| 60 | 148-149 | 60 | (E)-4-fluorostyryl-4-methoxybenzylsulfone |
| 61 | 176-177 | 66 | (E)-4-chlorostyryl-4-methoxybenzylsulfone |
| 62 | 179-181 | 60 | (E)-4-bromostyryl-4-methoxybenzylsulfone |
| 63 | 127-129 | 57 | (E)-2-nitrostyryl-4-methoxybenzylsulfone |
| 64 | 153-155 | 59 | (E)-3-nitrostyryl-4-methoxybenzylsulfone |
| 65 | 179-181 | 56 | (E)-4-nitrostyryl-4-methoxybenzylsulfone |
| 66 | 176-177 | 66 | (E)-4-chlorostyryl-4-nitrobenzylsulfone |
| 67 | 199-200 | 60 | (E)-4-fluorostyryl-4-nitrobenzylsulfone |

### Effect of (E)-Styryl Benzylsulfones on Breast, and Prostate Tumor Cell Lines

### A. Cells.

The effect of the (E)-styryl benzylsulfones on normal fibroblasts and on tumor cells of breast, and prostate origin was examined utilizing one or more of the following cell lines: breast tumor cell lines MCF-7 and BT-20; prostate tumor cell line DU-145; colorectal carcinoma cell line DLD-1; non-small cell lung carcinoma cell line H157; and NIH/3T3 and HFL cells. MCF-7 is estrogen-responsive, while BT-20 is an estrogen-unresponsive cell line. NIH/3T3 and HFL are normal murine and human fibroblasts, respectively. MCF-7, BT-20, DLD-1 and H157 were grown in Dulbecco's modified Eagle's medium (DMEM) containing 10% fetal bovine serum supplemented with penicillin and streptomycin. DU145 was cultured in RPMI with 10% fetal bovine serum containing penicillin and streptomycin. NIH3T3 and HFL cells were grown in DMEM containing 10% calf serum supplemented with penicillin and streptomycin. All cell cultures were maintained at 37°C in a humidified atmosphere of 5% CO₂.

### B. Treatment with (E)-Styryl Sulfones and Viability Assay

Cells were treated with test compound at 2.5 mM concentration and cell viability was determined after 96 hours by the Trypan blue exclusion method. The results are set forth in Table 2. Activity for each compound is reported as a range of cell induced death (% Death) with the lowest activity in the range of 5-10% and the highest being above 80%.

Normal cells HFL and NIH 3T3 were treated with the same compounds in Table 2 under the same conditions of concentration and time. The normal cells displayed 5% growth inhibition but no appreciable cell death.

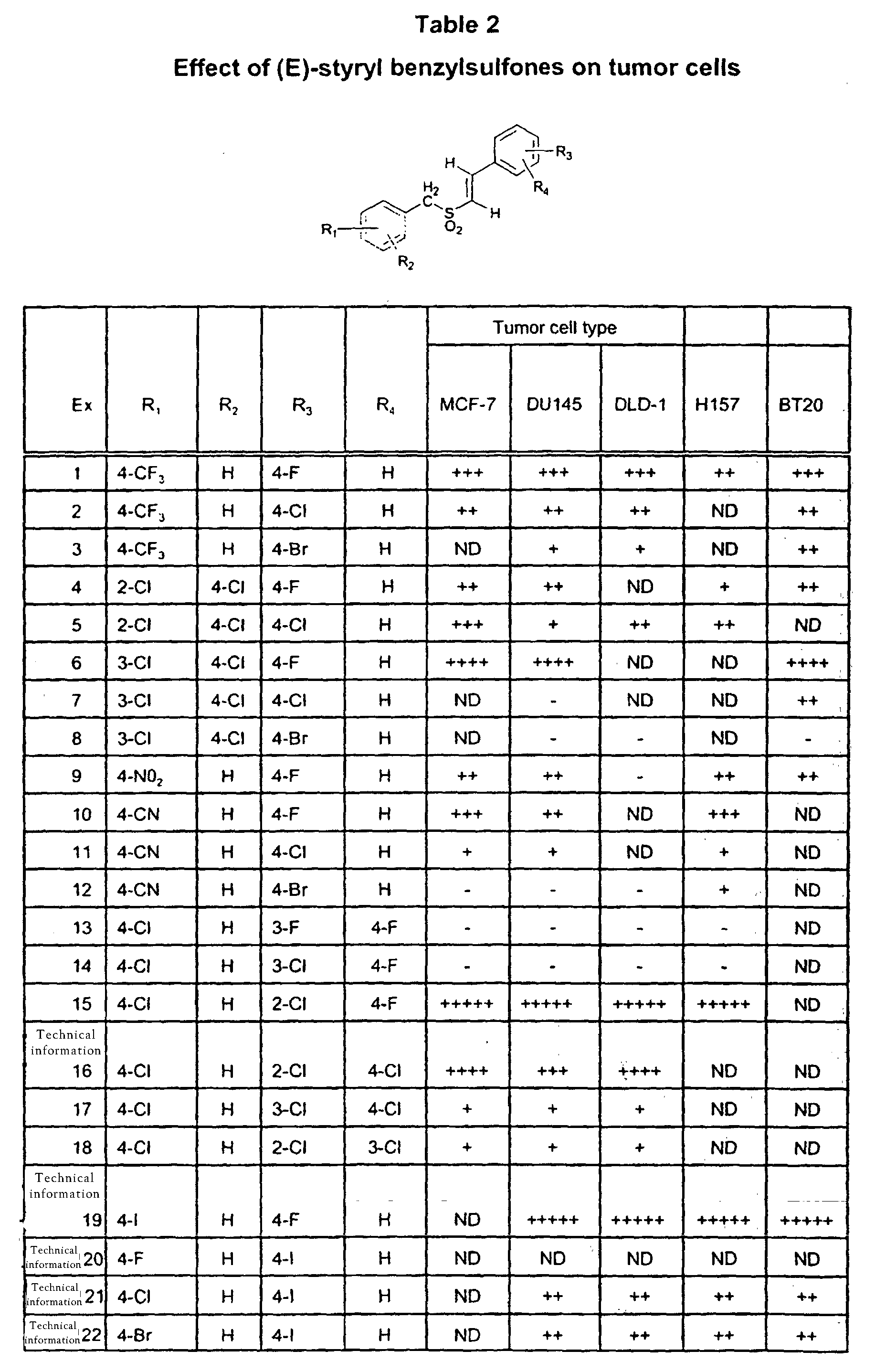

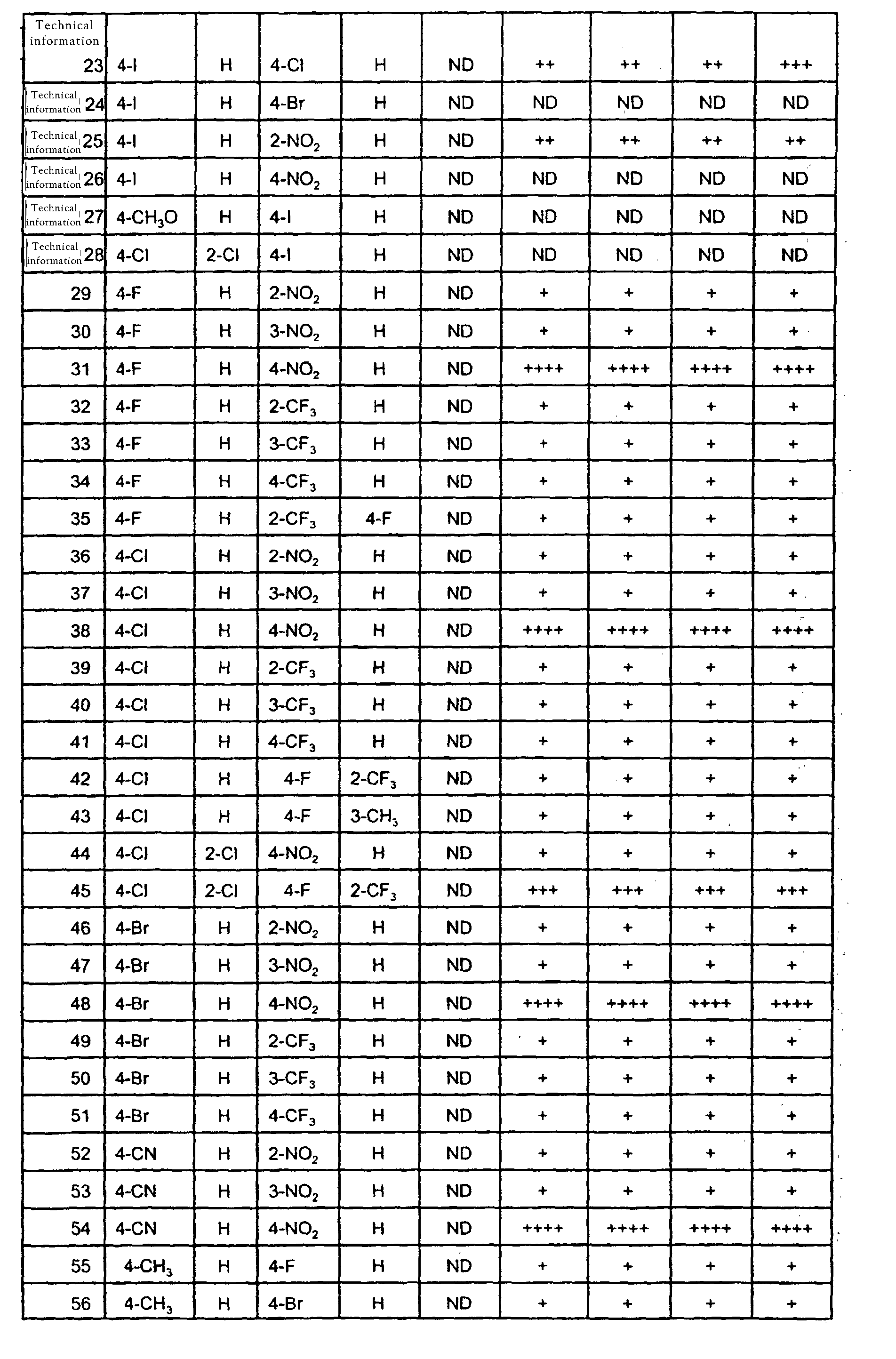

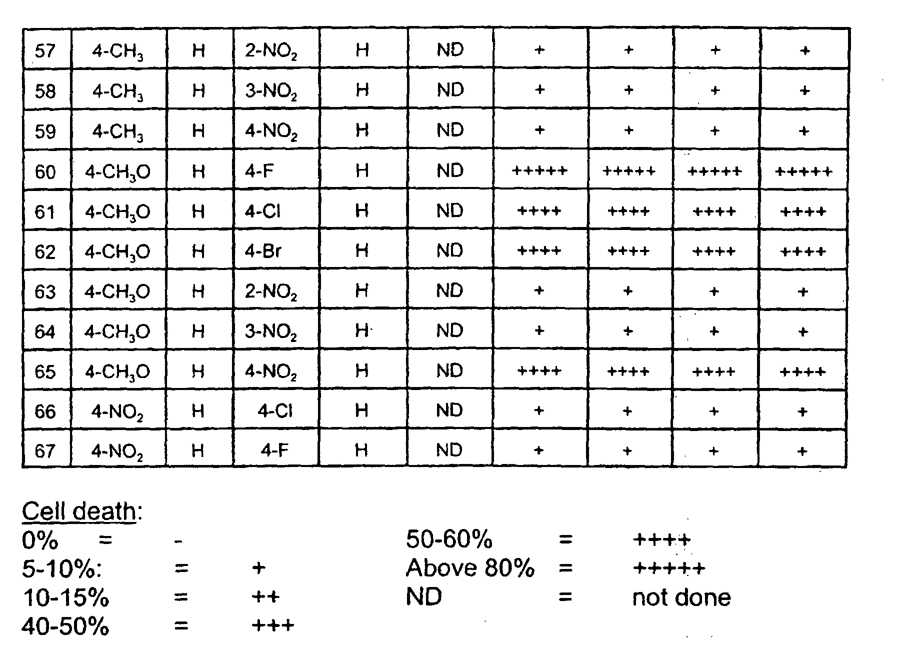

### Example 68

### Conjugation of (E)-4-Fluorostyryl 4-chlorobenzylsulfone to Keyhole Limpet Haemocyanin

A carboxylic acid derivative of (E)-4-fluorostyryl 4-chlorobenzylsulfone was synthesized by mixing 4-chlorobenzyl sulfonyl acetic acid (10 mmol), 4-fluorobenzaldehyde (10mmol), glacial acetic acid (15 ml) and piperidine (0.5 ml) at room temperature (22°C) over a magnetic stirrer for 5 hours. The mixture was then diluted with ether and the ethereal layer was washed with water. Evaporation of the ethereal layer yielded a semisolid material which on treating with 2-propanol gave a white solid. Recrystallization with 2-propanol gave 2-(4-chlorobenzyl sulfony)-3-(4-fluorophenyl) propenoic acid as white crystals, (yield 32%), m.p 111-112°C.

The above carboxylic acid derivative (10 mM) was made into an active ester by treatment with 10 mM 1-ethyl-3-(3-dimethylaminopropyl)carbodiimide hydrochloride (EDC) and 10 mM N-hydroxysuccinamide (NHS), and then cross-linked to KLH by mixing with 1 ml of a KLH water solution containing 500 mg KLH. The mixture was stirred at room temperature for 5-6 hours. The KLH conjugate was then separated by passing the mixture through a size exclusion column (PD 10, Pharmacia). The conjugate was then used to inject rabbits for raising antibodies.

## Claims

1. A compound of the formula: 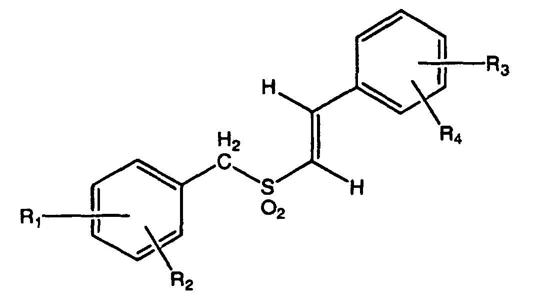 wherein:
R₁ and R₂ are independently selected from the group consisting of hydrogen, fluoro, chloro, bromo, C1-C6 alkyl, C1-C6 alkoxy, nitro, cyano and trifluoromethyl;
R₃ and R₄ are independently selected from the group consisting of hydrogen, fluoro, chloro, bromo, nitro, cyano and trifluoromethyl;
at least one of R₁ and R₂ is other than hydrogen; and
at least one of R₃ and R₄ is other than hydrogen;
with the proviso that
(a) when R₁ and R₃ are hydrogen and R₂ is 4-chloro, then R₄ may not be 4-chloro, 4-fluoro, 4-bromo, or 4-nitro;
(b) when R₁ and R₃ are hydrogen and R₂ is 4-fluoro or 4-bromo, then R₄ may not be 4-fluoro, 4-bromo, or 4-chloro;
(c) when R₁ and R₃ are hydrogen and R₂ is 4-nitro, then R₄ may not be 4-chloro, 4-nitro, 4-bromo, or 4-fluoro;
(d) when R₁ and R₃ are hydrogen and R₂ is 4-methyl, then R₄ may not be 4-chloro, 4-bromo, 4-fluoro, or 2-chloro;
(e) when R₁ is hydrogen, then R₂, R₃ and R₄ may not all be -fluoro;
(f) when R₁ is hydrogen and R₃ is 2-fluoro, then R₂ and R₄ may not both be selected from the group consisting of 4-chloro, 4-bromo and 4-fluoro; and
(g) the compound is not (E)-2-chloro-4-fluorostyryl-4-fluorobenzyl sulfone, (E)-2-chloro-4-fluorostyryl-4-chlorobenzyl sulfone, (E)-2-chloro-4-fluorostyryl-4-bromobenzyl sulfone, (E)-2,4-dichlorostyryl-4-fluorobenzyl sulfone, (E)-2,4-dichlorostyryl-4-chlorobenzyl sulfone, (E)-2,4-dichlorostyryl-4-bromobenzyl sulfone, (E)-2-chloro-4-bromostyryl-4-fluorobenzyl sulfone, (E)-2-chloro-4-bromostyryl-4-chlorobenzyl sulfone, or (E)-2-chloro-4-bromostyryl-4-bromobenzyl sulfone.

2. A compound according to claim 1 wherein at least one of R₁ and R₂ is located at the 2-, 3-or 4- position of the phenyl ring to which it is attached; and wherein at least one of R₃ and R₄ is located at the 2- or 4- position of the phenyl ring to which it is attached.

3. A compound according to claim 2 selected from the group consisting of (E)-4-fluorostyryl-4-trifluoromethylbenzylsulfone, (E)-2-trifluoromethyl-4-fluorostyryl-2,4-dichlorobenzylsulfone, (E)-4-fluorostyryl-4-cyanobenzylsulfone and (E)-4-fluorostyryl-4-nitrobenzylsulfone.

4. A compound according to claim 2 wherein at least one of R₁ and R₂ is chloro and at least one of R₃ and R₄ is chloro or fluoro.

5. A compound according to claim 4 selected from the group consisting of (E)-4-fluorostyryl-3,4-dichlorobenzylsulfone, (E)-4-fluorostyryl-2,4-dichlorobenzylsulfone, (E)-2-chloro-4-fluorostyryl-4-chlorobenzylsulfone and (E)-2,4-dichlorostyryl-4-chlorobenzylsulfone.

6. A compound according to claim 2 wherein R₂ is 4-halogen or 4-cyano, and R₄ is 4-nitro.

7. A compound according to claim 6 selected from the group consisting of (E)-4-nitrostyryl-4-fluorobenzylsulfone, (E)-4-nitrostyryl-4-bromobenzylsulfone and (E)-4-nitrostyryl-4-cyanobenzylsulfone.

8. A compound according to claim 2 wherein R₂ is 4-C1-C6 alkoxy, and R₄ is 4-halogen or 4-nitro.

9. A compound according to claim 8 selected from the group consisting of (E)-4-fluorostyryl-4-methoxybenzylsulfone, (E)-4-chlorostyryl-4-methoxybenzylsulfone, (E)-4-bromostyryl-4-methoxybenzylsulfone and (E)-4-nitrostyryl-4-methoxybenzylsulfone.

10. A compound for use in medicine of the formula: 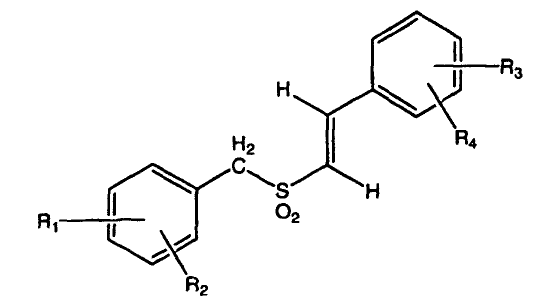 wherein:
R₁ and R₂ are independently selected from the group consisting of hydrogen, fluoro, chloro, bromo, C1-C6 alkyl, C1-C6 alkoxy, nitro, cyano and trifluoromethyl;
R₃ and R₄ are independently selected from the group consisting of hydrogen, fluoro, chloro, bromo, nitro, cyano and trifluoromethyl;
at least one of R₁ and R₂ is other than hydrogen; and at least one of R₃ and R₄ is other than hydrogen;
with the proviso that
(a) when R₁ and R₃ are hydrogen and R₂ is 4-bromo or 4-chloro, R₄ may not be 4-chloro, 4-fluoro, or 4-bromo;
(b) when R₁ and R₃ are hydrogen and R₂ is 4-fluoro, R₄ may not be 4-fluoro or 4-bromo;
(c) when R₁ is hydrogen and R₄ is 2-fluoro, then R₂ and R₃ may not be 4-fluoro;
(d) when R₁ is hydrogen and R₃ is 4-hydrogen, 4-chloro, or 4-bromo, and R₄ is 2-hydrogen, 2-chloro or 2-fluoro, then R₂ may not be 4-hydrogen, 4-chloro, 4-fluoro, or 4-bromo; and
(e) the compound is not (*E*)-2-chloro-4-fluorostyryl-4-fluorobenzyl sulfone, (*E*)-2-chloro-4-fluorostyryl-4-chlorobenzyl sulfone, (*E*)-2-chloro-4-fluorostyryl-4-bromobenzyl sulfone, (*E*)-2,4-difluorostyryl-4-chlorobenzyl sulfone, or (*E*)-2,4-difluorostyryl-4-bromobenzyl sulfone.

11. A pharmaceutical composition comprising a pharmaceutically acceptable carrier and a compound of any preceding claim.

12. Use of a compound according to any of claims 1 to 10 in the preparation of a medicament for treatment of a proliferative disorder.

13. Use of a compound according to claim 12 wherein the proliferative disorder is selected from the group consisting of cancer, hemangiomatosis in new born, secondary progressive multiple sclerosis, chronic progressive myelodegenerative disease, neurofibromatosis, ganglioneuromatosis, keloid formation, Pagets Disease of the bone, fibrocystic disease of the breast, Peronies and Duputren's fibrosis, restenosis and cirrhosis.

14. Use of a compound according to claim 13 wherein the proliferative disorder is a cancer selected from the group consisting of ovarian, breast, prostate, lung, renal, colorectal and brain cancers, or the cancer is a leukaemia.

15. Use of a compound according to any of claims 1 to 10 in the preparation of a medicament for inducing apoptosis of tumor cells in an individual afflicted with cancer.

16. Use of a compound according to claim 15 wherein the tumor cells are selected from the group consisting of ovarian, breast, prostate, lung, colorectal, renal and brain tumors.

17. A compound useful as an intermediate in the synthesis of a compound according to claim 1, said intermediate compound having the formula 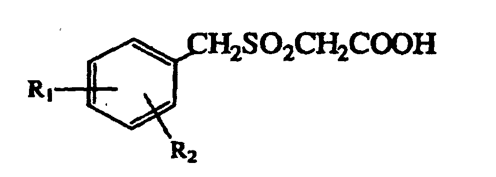 wherein:
R₁ and R₂ are both halogen; or
R₁ is hydrogen and R₂ is CF₃;
or a salt thereof, provided that the intermediate compound is other than 2,4-dichlorobenzylsulfonylacetic acid

18. A compound according to claim 17 wherein R₁ and R₂ are both chloro.

19. A compound according to claim 18 selected from the group consisting of 4-trifluoromethylbenzylsulfonyl acetic acid, 2,4-dichlorobenzylsulfonyl acetic acid and 3,4-dichlorobenzylsulfonyl acetic acid, and salts thereof.

## Patentansprüche

1. Eine Verbindung mit folgender Formel: 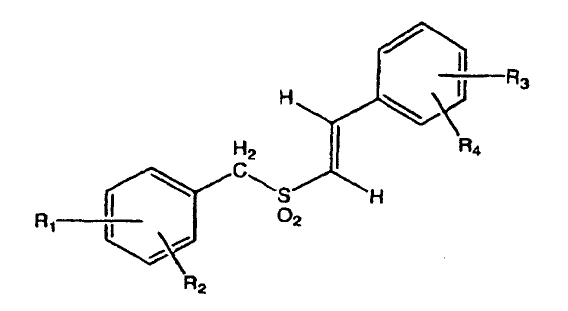 wobei:
R₁ und R₂ unabhängig aus der Gruppe, bestehend aus Wasserstoff, Fluor, Chlor, Brom, C1-C6-Alkyl, C1-C6-Alkoxy, Nitro, Cyan und Trifluormethyl, ausgewählt sind;
R₃ und R₄ unabhängig aus der Gruppe, bestehend aus Wasserstoff, Fluor, Chlor, Brom, Nitro, Cyan und Trifluormethyl ausgewählt sind; wobei mindestens entweder R₁ oder R₂ nicht Wasserstoff ist; und
mindestens entweder R₃ oder R₄ nicht Wasserstoff ist; mit der Maßgabe, dass
(a) wenn R₁ und R₃ Wasserstoff sind und R₂ 4-Chlor ist, dann R₄ nicht 4-Chlor, 4-Fluor, 4-Brom oder 4-Nitro sein kann;
(b) wenn R₁ und R₃ Wasserstoff sind und R₂ 4-Fluor oder 4-Brom ist, dann R₄ nicht 4-Fluor, 4-Brom oder 4-Chlor sein kann;
(c) wenn R₁ und R₃ Wasserstoff sind und R₂ 4-Nitro ist, dann R₄ nicht 4-Chlor, 4-Nitro, 4-Brom oder 4-Fluor sein kann;
(d) wenn R₁ und R₃ Wasserstoff sind und R₂ 4-Methyl ist, dann R₄ nicht 4-Chlor, 4-Brom, 4-Fluor oder 2-Chlor sein kann;
(e) wenn R₁ Wasserstoff ist, dann R₂, R₃ und R₄ nicht alle -Fluor sein können;
(f) wenn R₁ Wasserstoff ist und R₃ 2-Fluor ist, dann R₂ und R₄ nicht beide aus der Gruppe, bestehend aus 4-Chlor, 4-Brom und 4-Fluor, ausgewählt werden können; und
(g) die Verbindung nicht (E)-2-Chlor-4-fluorstyryl-4fluorbenzylsulfon, (E)-2-Chlor-4-fluorstyryl-4chlorbenzylsulfon, (E)-2-Chlor-4-fluorstyryl-4brombenzylsulfon, (E)-2, 4-Dichlorstyryl-4-fluorbenzylsulfon, (E)-2, 4-Dichlorstyryl-4-chlorbenzylsulfon, (E)-2, 4-Dichlorstyryl-4-brombenzylsulfon, (E)-2-Chlor-4-bromstyryl-4fluorbenzylsulfon, (E)-2-Chlor-4-bromstyryl-4chlorbenzylsulfon oder (E)-2-Chlor-4-bromstyryl-4brombenzylsulfon ist.

2. Verbindung gemäß Anspruch 1, wobei mindestens entweder R₁ oder R₂ an der 2-, 3- oder 4-Position des Phenylrings, an dem es befestigt ist, befindlich ist; und wobei mindestens entweder R₃ oder R₄ an der 2- oder 4-Position des Phenylrings, an dem es befestigt ist, befindlich ist.

3. Verbindung gemäß Anspruch 2, die aus der Gruppe, bestehend aus (E)-4-Fluorstyryl-4-trifluormethylbenzylsulfon, (E)-2-Trifluormethyl-4-fluorstyryl-2,4-dichlorbenzylsulfon, (E)-4-Fluorstyryl-4cyanbenzylsulfon und (E)-4-Fluorstyryl-4-nitrobenzylsulfon, ausgewählt ist.

4. Verbindung gemäß Anspruch 2, wobei mindestens entweder R₁ oder R₂ Chlor und mindestens entweder R₃ oder R₄ Chlor oder Fluor ist.

5. Verbindung gemäß Anspruch 4, die aus der Gruppe, bestehend aus (E)-4-Fluorstyryl-3,4-dichlorbenzylsulfon, (E)-4-Fluorstyryl-2,4dichlorbenzylsulfon, (E)-2-Chlor-4-fluorstyryl-4-chlorbenzylsulfon und (E)-2,4-Dichlorstyryl-4-chlorbenzylsulfon, ausgewählt ist.

6. Verbindung gemäß Anspruch 2, wobei R₂ ein 4-Halogen oder 4-Cyan ist und R₄ 4-Nitro ist.

7. Verbindung gemäß Anspruch 6, die aus der Gruppe, bestehend aus (E)-4-Nitrostyryl-4-fluorbenzylsulfon, (E)-4-Nitrostyryl-4brombenzylsulfon und (E)-4-Nitrostyryl-4-cyanbenzylsulfon, ausgewählt ist.

8. Verbindung gemäß Anspruch 2, wobei R₂ ein 4-C1-C6-Alkoxy und R₄ 4-Halogen oder 4-Nitro ist.

9. Verbindung gemäß Anspruch 8, die aus der Gruppe, bestehend aus (E)-4-Fluorstyryl-4-methoxybenzylsulfon, (E)-4-Chlorstyryl-4-methoxybenzylsulfon, (E)-4-Bromstyryl-4-methoxybenzylsulfon und (E)-4-Nitrostyryl-4-methoxybenzylsulfon, ausgewählt ist.

10. Eine Verbindung zur Verwendung in der Medizin mit der folgenden Formel: 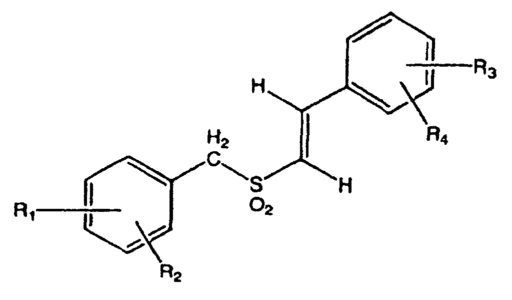 wobei:
R₁ und R₂ unabhängig aus der Gruppe, bestehend aus Wasserstoff, Fluor, Chlor, Brom, C1-C6-Alkyl, C1-C6-Alkoxy, Nitro, Cyan und Trifluormethyl, ausgewählt sind;
R₃ und R₄ unabhängig aus der Gruppe, bestehend aus Wasserstoff, Fluor, Chlor, Brom, Nitro, Cyan und Trifluormethyl, ausgewählt sind;
mindestens R₁ oder R₂ nicht Wasserstoff ist; und mindestens entweder R₃ oder R₄ nicht Wasserstoff ist;
mit der Maßgabe, dass
(a) wenn R₁ und R₃ Wasserstoff sind und R₂ 4-Brom oder 4-Chlor ist, dann R₄ nicht 4-Chlor, 4-Fluor oder 4-Brom sein kann;
(b) wenn R₁ und R₃ Wasserstoff sind und R₂ 4-Fluor ist, dann R₄ nicht 4-Fluor oder 4-Brom sein kann;
(c) wenn R₁ Wasserstoff ist und R₄ 2-Fluor ist, dann R₂ und R₃ nicht 4-Fluor sein können;
(d) wenn R₁ Wasserstoff ist und R₃ 4-Wasserstoff, 4-Chlor oder 4-Brom ist und R₄ 2-Wasserstoff, 2-Chlor oder 2-Fluor ist, dann R₂ nicht 4-Wasserstoff, 4-Chlor, 4-Fluor oder 4-Brom sein kann; und
(e) die Verbindung nicht (*E*)-2-Chlor-4-fluorstyryl-4-fluorbenzylsulfon, (*E*)-2-Chlor-4-fluorstyryl-4-chlorbenzylsulfon, (*E*)-2-Chlor-4-fluorstyryl-4-brombenzylsulfon, (*E*)*-*2,4-Difluorstyryl-4-chlorbenzylsulfon oder (*E*)-2,4-Difluorstyryl-4-brombenzylsulfon ist.

11. Eine pharmazeutische Zusammensetzung, die einen pharmazeutisch akzeptablen Träger und eine Verbindung gemäß einem der vorhergehenden Ansprüche beinhaltet.

12. Die Verwendung einer Verbindung gemäß einem der Ansprüche 1 bis 10 bei der Präparation eines Medikaments zur Behandlung einer proliferativen Störung.

13. Verwendung einer Verbindung gemäß Anspruch 12, wobei die proliferative Störung aus der Gruppe, bestehend aus Krebs, Haemangiomatosis bei Neugeborenen, sekundärer progressiver Multipler Sklerose, chronisch progressiver myeloder generativer Störung, Neurofibromatosis generalisata, Ganglioneuromatose, Keloidbildung, Osteodystrophia deformans, proliferierender Mastopathie, Peyronie- und Dupuytren-Krankheit, Restenose und Zirrhose, ausgewählt ist.

14. Verwendung einer Verbindung gemäß Anspruch 13, wobei die proliferative Störung ein Krebs aus der Gruppe, bestehend aus Eierstock-, Brust-, Prostata-, Lungen-, Nieren-, kolorektalem und Gehirnkrebs, ausgewählt ist oder der Krebs Leukämie ist.

15. Verwendung einer Verbindung gemäß einem der Ansprüche 1 bis 10 bei der Präparation eines Medikaments zur Induzierung der Apoptose von Tumorzellen bei einem an Krebs leidenden Patienten.

16. Verwendung einer Verbindung gemäß Anspruch 15, wobei die Tumorzellen aus der Gruppe, bestehend aus Eierstock-, Brust-, Prostata-, Lungen-, kolorektalen, Nieren- und Gehirntumoren, ausgewählt sind.

17. Eine Verbindung, die bei der Synthese einer Verbindung gemäß Anspruch 1 als Zwischenprodukt nützlich ist, wobei die Zwischenproduktverbindung folgende Formel aufweist: 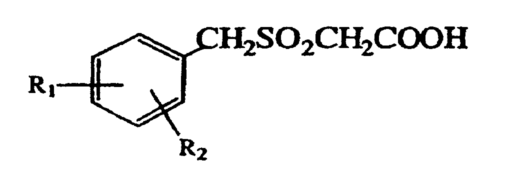 wobei:
R₁ und R₂ beide Halogen sind; oder
R₁ Wasserstoff ist und R₂ CF₃ ist;
oder ein Salz davon, vorausgesetzt, dass die Zwischenproduktverbindung nicht 2,4-Dichlorbenzylsulfonylessigsäure ist.

18. Verbindung gemäß Anspruch 17, wobei R₁ und R₂ beide Chlor sind.

19. Verbindung gemäß Anspruch 18, die aus der Gruppe, bestehend aus 4-Trifluormethylbenzylsulfonylessigsäure, 2,4-Dichlorbenzylsulfonylessigsäure und 3,4-Dichlorbenzylsulfonylessigsäure und deren Salze, ausgewählt ist.

## Revendications

1. Un composé de formule : 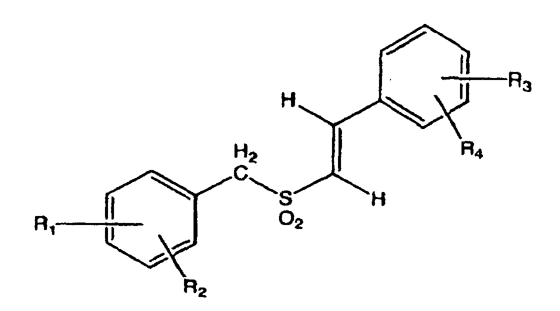 dans lequel :
R₁ et R₂ sont indépendamment sélectionnés dans le groupe consistant en hydrogène, fluoro, chloro, bromo, alkyl C1-C6, alkoxy C1-C6, nitro, cyano et trifluorométhyl ;
R₃ et R₄ sont indépendamment sélectionnés dans le groupe consistant en hydrogène, fluoro, chloro, bromo, nitro, cyano et trifluorométhyl ; au moins soit R₁, soit R₂ est autre que hydrogène ; et
au moins soit R₃, soit R₄ est autre que hydrogène ;
à condition que
(a) lorsque R₁ et R₃ sont hydrogène et R₂ est 4-chloro, R₄ ne puisse alors pas être 4-chloro, 4-fluoro, 4-bromo, ou 4-nitro ;
(b) lorsque R₁ et R₃ sont hydrogène et R₂ est 4-fluoro ou 4-bromo, R₄ ne puisse alors pas être 4-fluoro, 4-bromo, ou 4-chloro ;
(c) lorsque R₁ et R₃ sont hydrogène et R₂ est 4-nitro, R₄ ne puisse alors pas être 4-chloro, 4-nitro, 4-bromo, ou 4-fluoro ;
(d) lorsque R₁ et R₃ sont hydrogène et R₂ est 4-méthyl, R₄ ne puisse alors pas être 4-chloro, 4-bromo, 4-fluoro, ou 2-chloro ;
(e) lorsque R₁ est hydrogène, R₂, R₃ et R₄ ne puissent alors pas tous être -fluoro ;
(f) lorsque R₁ est hydrogène et R₃ est 2-fluoro, R₂ et R₄ ne puissent alors pas être tous deux sélectionnés dans le groupe consistant en 4-chloro, 4-bromo et 4-fluoro ; et que
(g) le composé ne soit pas (E)-2-chloro-4-fluorostyryl-4-fluorobenzylsulfone, (E)-2-chloro-4-fluorostyryl-4-chlorobenzylsulfone, (E)-2-chloro-4-fluorostyryl-4-bromobenzylsulfone, (E)-2, 4-dichlorostyryl-4-fluorobenzylsulfone, (E)-2, 4-dichlorostyryl-4-chlorobenzylsulfone, (E)-2, 4-dichlorostyryl-4-bromobenzylsulfone, (E)-2-chloro-4-bromostyryl-4-fluorobenzylsulfone, (E)-2-chloro-4-bromostyryl-4-chlorobenzylsulfone, ou (E)-2-chloro-4-bromostyryl-4-bromobenzylsulfone.

2. Un composé selon la revendication 1 dans lequel au moins soit R₁, soit R₂ est situé à la position 2-, 3- ou 4- du cycle phénylique auquel il est attaché ; et dans lequel au moins soit R₃, soit R₄ est situé à la position 2- ou 4- du cycle phénylique auquel il est attaché.

3. Un composé selon la revendication 2 sélectionné dans le groupe consistant en (E)-4-fluorostyryl-4-trifluorométhylbenzylsulfone, (E)-2-trifluorométhyl-4-fluorostyryl-2, 4-dichlorobenzylsulfone, (E)-4-fluorostyryl-4-cyanobenzylsulfone et (E)-4-fluorostyryl-4-nitrobenzylsulfone.

4. Un composé selon la revendication 2 dans lequel au moins soit R₁, soit R₂ est chloro et au moins soit R₃, soit R₄ est chloro ou fluoro.

5. Un composé selon la revendication 4 sélectionné dans le groupe consistant en (E)-4-fluorostyryl-3, 4-dichlorobenzylsulfone, (E)-4-fluorostyryl-2, 4-dichlorobenzylsulfone, (E)-2-chloro-4-fluorostyryl-4-chlorobenzylsulfone et (E)-2, 4-dichlorostyryl-4-chlorobenzylsulfone.

6. Un composé selon la revendication 2 dans lequel R₂ est 4-halogène ou 4-cyano et R₄ est 4-nitro.

7. Un composé selon la revendication 6 sélectionné dans le groupe consistant en (E)-4-nitrostyryl-4-fluorobenzylsulfone, (E)-4-nitrostyryl-4-bromobenzylsulfone et (E)-4-nitrostyryl-4-cyanobenzylsulfone.

8. Un composé selon la revendication 2 dans lequel R₂ est alkoxy 4-C1-C6, et R₄ est 4-halogène ou 4-nitro.

9. Un composé selon la revendication 8 sélectionné dans le groupe consistant en (E)-4-fluorostyryl-4-méthoxybenzylsulfone, (E)-4-chlorostyryl-4-méthoxybenzylsulfone, (E)-4-bromostyryl-4-méthoxybenzylsulfone et (E)-4-nitrostyryl-4-méthoxybenzylsulfone.

10. Un composé destiné à être utilisé en médecine de formule : 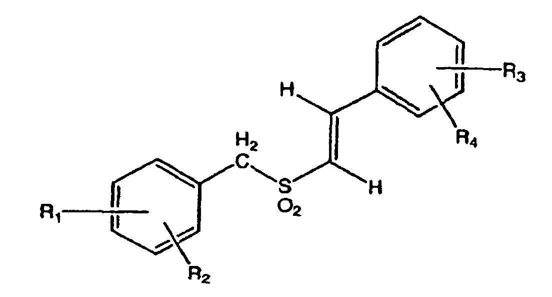 dans lequel :
R₁ et R₂ sont indépendamment sélectionnés dans le groupe consistant en hydrogène, fluoro, chloro, bromo, alkyl C1-C6, alkoxy C1-C6, nitro, cyano et trifluorométhyl ;
R₃ et R₄ sont indépendamment sélectionnés dans le groupe consistant en hydrogène, fluoro, chloro, bromo, nitro, cyano et trifluorométhyl ;
au moins soit R₁, soit R₂ est autre que hydrogène ; et au moins soit R₃, soit R₄ est autre que hydrogène ;
à condition que
(a) lorsque R₁ et R₃ sont hydrogène et R₂ est 4-bromo ou 4-chloro, R₄ ne puisse pas être 4-chloro, 4-fluoro, ou 4-bromo ;
(b) lorsque R₁ et R₃ sont hydrogène et R₂ est 4-fluoro, R₄ ne puisse pas être 4-fluoro ou 4-bromo ;
(c) lorsque R₁ est hydrogène et R₄ est 2-fluoro, R₂ et R₃ ne puissent alors pas être 4-fluoro ;
(d) lorsque R₁ est hydrogène et R₃ est 4-hydrogène, 4-chloro, ou 4-bromo, et R₄ est 2-hydrogène, 2-chloro ou 2-fluoro, R₂ ne puisse alors pas être 4-hydrogène, 4-chloro, 4-fluoro, ou 4-bromo ; et
(e) le composé ne soit pas (*E*)-2-chloro-4-fluorostyryl-4-fluorobenzylsulfone, (*E*)-2-chloro-4-fluorostyryl-4-chlorobenzylsulfone, (*E*)-2-chloro-4-fluorostyryl-4-bromobenzylsulfone, (*E*)-2, 4-difluorostyryl-4-chlorobenzylsulfone ou (*E*)-2, 4-difluorostyryl-4-bromobenzylsulfone.

11. Une composition pharmaceutique comportant un support acceptable d'un point de vue pharmaceutique et un composé de n'importe quelle revendication précédente.

12. Utilisation d'un composé selon n'importe lesquelles des revendications 1 à 10 dans la préparation d'un médicament destiné au traitement d'un trouble prolifératif.

13. Utilisation d'un composé selon la revendication 12 dans lequel le trouble prolifératif est sélectionné dans le groupe consistant en cancer, hémangiomatose chez le nouveau-né, sclérose en plaques progressive secondaire, maladie myélodégénérative progressive chronique, neurofibromatose, ganglioneuromatose, formation de chéloïdes, maladie osseuse de Paget, maladie fibrokystique du sein, fibroses de Peyronie et de Dupuytren, resténose et cirrhose.

14. Utilisation d'un composé selon la revendication 13 dans lequel le trouble prolifératif est un cancer sélectionné dans le groupe consistant en cancers ovarien, du sein, de la prostate, du poumon, rénal, colorectal et du cerveau, ou le cancer est une leucémie.

15. Utilisation d'un composé selon n'importe lesquelles des revendications 1 à 10 dans la préparation d'un médicament destiné à provoquer l'apoptose de cellules tumorales chez un individu souffrant de cancer.

16. Utilisation d'un composé selon la revendication 15 dans laquelle les cellules tumorales sont sélectionnées dans le groupe consistant en tumeurs ovarienne, du sein, de la prostate, du poumon, colorectale, rénale et du cerveau.

17. Un composé utile en tant qu'intermédiaire dans la synthèse d'un composé selon la revendication 1, ledit composé intermédiaire ayant la formule 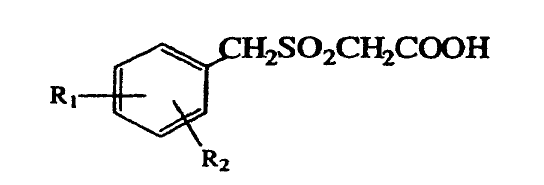 dans lequel :
R₁ et R₂ sont tous deux halogène ; ou
R₁ est hydrogène et R₂ est CF₃ ;
ou un sel de celui-ci, à condition que le composé intermédiaire soit autre que l'acide 2, 4-dichlorobenzylsulfonylacétique.

18. Un composé selon la revendication 17 dans lequel R₁ et R₂ sont tous deux chloro.

19. Un composé selon la revendication 18 sélectionné dans le groupe consistant en acide 4-trifluorométhylbenzylsulfonylacétique, acide 2, 4-dichlorobenzylsulfonylacétique et acide 3, 4-dichlorobenzylsulfonylacétique, et sels de ceux-ci.
